Europäisches Patentamt

European Patent Office

Office européen des brevets

(11) Publication number: **0 059 218**

**B1**

(12) **EUROPEAN PATENT SPECIFICATION**

(45) Date of publication of patent specification: **10.06.87**

(21) Application number: **81902864.8**

(22) Date of filing: **09.09.81**

(86) International application number:
**PCT/US81/01208**

(87) International publication number:
**WO 82/00754 18.03.82 Gazette 82/08**

(51) Int. Cl.⁴: **A 61 B 17/42,** A 61 M 25/00,
A 61 M 37/02

(54) **APPARATUS FOR ARTIFICIAL EMBRYONATION AND TISSUE RECOVERY.**

(30) Priority: **09.09.80 US 185877**
**22.12.80 US 219014**

(43) Date of publication of application:
**08.09.82 Bulletin 82/36**

(45) Publication of the grant of the patent:
**10.06.87 Bulletin 87/24**

(84) Designated Contracting States:
**AT CH DE FR GB LI LU NL SE**

(56) References cited:
**US-A-2 845 930**
**US-A-3 095 871**
**US-A-3 636 940**
**US-A-3 687 806**
**US-A-3 811 423**
**US-A-3 811 424**
**US-A-3 854 470**
**US-A-4 004 588**
**US-A-4 178 936**

(73) Proprietor: **FERTILITY AND GENETICS**
**RESEARCH, INC.**
**135 South LaSalle Street**
**Chicago Illinois 60603 (US)**

(72) Inventor: **Seed, Richard G.**
**740 Belleforte**
**Oak Park, IL 60302 (US)**

(74) Representative: **Patentanwälte Schaumburg &**
**Thoenes**
**Mauerkircherstrasse 31 Postfach 86 07 48**
**D-8000 München 80 (DE)**

Courier Press, Leamington Spa, England.

## Description

### Background of the invention
### 1. Field of the invention

The invention relates to a uterine catheter for use in embryo transfer and tissue recovery processes and the apparatus therefor, and particularly to artificial embryonation and like methods which are useful for non-surgical recovery of eggs and other internal materials from a patient. Artificial embryonation is a term I have coined to define the disclosed transplant of a fertilized egg from a volunteer fertile donor to an infertile recipient. Non-surgical recovery and transfer tools are disclosed which enable this new process to be performed, as well as new uses of known compounds which enable this process to be performed for human patients.

### 2. Description of the prior art

While embryo transfer has been performed in several species during the last decade, making practical commercial use by way of substantial improvements to ideas dating back to 1893 in farm animals, only recent improvements have made the techniques possible on other than complicated experimental bases. Non-surgical techniques have not existed for transfers which do not involve rectal palpation, nor have they been possible for humans, one of those species which does not allow for such palpation. In addition, steps useful in one species have not proven to be susceptible to general usage in other species due to a variety of factors, including lack of tools enabling more general usage and the lack of responsiveness to various compounds and media.

Surgical recovery and fertilization of a human egg in vitro does appear to have been first accomplished in England in 1969 by physiologists R. G. Edwards and B. D. Bavister, and gynecologist P. C. Steptoe, by techniques and methods not fully disclosed. The claim to the birth of Louise Joy Brown on July 25, 1978, was the first recorded case in which a human egg had been surgically taken from a woman's ovary, fertilized externally and then implanted in the same woman's uterus, where it developed to term. The leading and pioneer patents in the field may be reviewed in U.S. Class 128/1R and International Class A61k 17/100, and in the references cited therein and in the literature therein referenced, the state of the art relating to farm animals can be determined. In addition thereto, reference may be had to the serial, *Theriogenology*, published by Geron-X, Inc. Los Altos, California, *Science, Journal of Reproduction and Fertility*, and *The Biology of Reproduction*. The work of L. E. A. Rowsen and his associates at the A. R. C. Unit of Reproductive Physiology and Biochemistry, Animal Research Station, Cambridge, England is reported in these serials.

U.S. Patent 3,854,470 issued December 17, 1974 to *Augspurger*, and *Bovine Embryo Transfer Procedures*, Colorado State University Animal Reproduction Laboratory, General Series 975 (1978, 1980) represent a comprehensive disclosure of present practice, particularly as it relates to cattle, and *Gravlee*, U.S. Patent No. 3,527,203, issued August 4, 1970 discloses an irrigating device for collecting tissue samples from the uterus, an *Hasson*, U.S. Patent No. 3,948,270 issued April 6, 1976 discloses a uterin cannula with a rigid tube inside a Foley Catheter, similar to that used in non-surgical cattle embryo recovery.

*Brown*, U.S. Patent No. 2,845,930 refers to an apparatus for determining the patency of the uterus in cows and similar animals. *Newcomb*, U.S. Patent No. 4,178,936 and *Alexander*, U.S. Patent No. 4,004,588 refer to apparatus for collecting and flushing ova from animals having uterine horns. *Mann*, U.S. Patent No. 3,095,871 refers to an apparatus for determining characteristics of the human uterus. Each of these apparatus includes a rigid introducing member.

In addition, *Science*, Vol. 192, pp. 1246—1247 reported the surgical transfer of a naturally inseminated Papio cyneocephalus embryo to a naturally synchronized recipient. U.S. Patent 4,193,392 of March 19, 1980 to *Barnett* discloses a surgical method of ova recovery in animals such as cattle.

Without detracting from the considerable reported accomplishments, it is believed that those skilled in the art appreciate that the adoption of the prior art techniques which are scattered throughout numerous sources will not yield the results reported herein, nor lead one to the novel apparatus, the new uses of known compounds, not to the method I have used to achieve artificial embryonation and the other methods for non-surgical recovery of eggs and tissue from a subject. While my apparatus is adapted to use with many mammals, and some of my methods may be employed therewith, I am particularly concerned with the problems of infertility among human couples.

It has been estimated that one-third of the infertility in the United States results from disease of the Fallopian tubes, and that as many as 10 percent of all couples are infertile. In approximately 1.5 percent of all couples, both partners are infertile. The gross estimates of the number of couples so affected has been estimated to be between 500,000 and 1,000,000. In perhaps one-third of these infertile couples, the female might have a uterus capable of accepting an embryo and carrying it to term.

Due to the twin forces of birth control and legalized abortion, the number of children available for adoption by these infertile couples has decreased dramatically. Without an alternate method, this leaves large numbers of childless couples destined to remain that way. People who have children or who elect not to have children can scarcely understand the levels of frustration, anguish and despair that many of these couples feel.

## Summary of the invention

The present invention provides a uterine catheter enabling non-surgical recovery and transfer of ova from mammalian subjects which are not suitable for manual palpation. In addition, the catheter provides unique, dual purpose capabilities as a tool for various types of reproductive work, such as uterine recovery of tissue including ova for tissue culture.

The present invention provides a new, and unique approach to the synchronization of the particular donor and recipient through the use of known compounds, which in other species has shown adverse side effects and has been abandoned.

The catheter of the invention allows for non-surgical transfer in smaller mammals which cannot be rectally palpated with the hand, and is particularly suitable for those of the threatened species and smaller domestic mammals, and particularly the primates and humans.

The present invention enables the achievement of artificial embryonation, as described.

It will be understood that many combinations of methods described may be made, and while this introduction could be elaborated here, I feel it better to discuss may invention in greater detail below so that a greater understanding may be had by the reader.

Use of the disclosure made in the description and claims hereto, which I now make, may be subject to various combinations, modifications and rearrangements which will occur to those skilled in the art, both now, and in the future, without departing from the scope of the claims appended hereto. The generally adaptable apparatus, and the special uses which I have made of known compounds, are believed to represent substantial advances in the art, as well as various methods, and the modifications thereto which I disclose.

Accordingly, my disclosure will describe first the referenced drawings.

## Brief description of the drawings

Figure 1 is a longitudinal cross-section in a medial plane through a female human figure showing a two-lumen uterine catheter in place in the uterus.

Figure 2 is an elevational view of a single lumen uterine catheter.

Figure 3 is an enlarged transverse cross-sectional view on line 3—3 of Figure 2.

Figure 4 is an elevational view of a two-lumen (double-lumen) uterine catheter.

Figure 5 is an enlarged transverse cross-sectional view on line 5—5 of Figure 4.

Figure 6 is an enlarged transverse cross-sectional view taken on line 6—6 of Figure 4.

Figure 7 is an enlarged end view as shown along line 7—7 of Figure 4.

Figure 8 is an elevational view of a three-lumen (triple lumen) uterine catheter.

Figure 9 is an enlarged transverse cross-sectional view taken on line 9—9 of Figure 8.

Figure 10 is an enlarged transverse cross-sectional view taken on line 10—10 of Figure 8.

Figure 11 is an enlarged transverse cross-sectional view taken on line 11—11 of Figure 8.

Figure 12 is an enlarged elevated view taken on line 12—12 of Figure 8.

Figure 13 is an enlarged fragmented sectional view of the cuff portion in inflated condition.

## Detailed description of the drawings

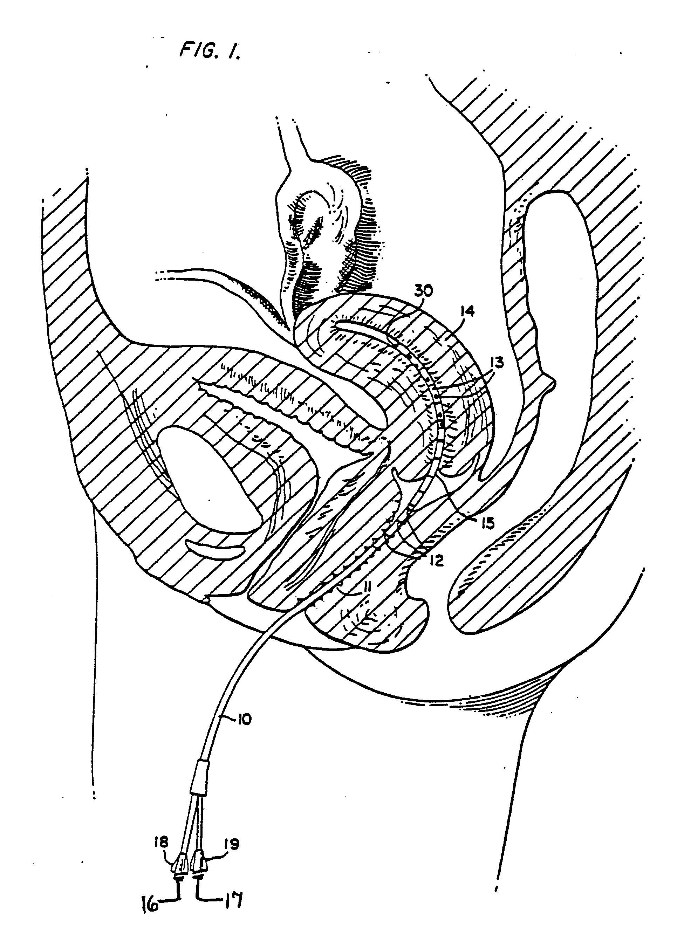

Figure 1 shows a double lumen uterine catheter 10 in place in a uterus 14, having been inserted through the cervical os 15 through the vagina 11. Markings 12 may be placed along the length of the catheter at predetermined intervals in order to determine how far into the uterus the uterine catheter has been placed. Liquid may be introduced into the uterus through the distal end of lumen 16, flowing out of port 30 into the uterus. Adapter 18 may be directly connected to a source of liquid. Liquid may be withdrawn from the uterus through ports 13, and allowed to flow through lumen 17. Adapter 19 may be directly connected to a source of suction to aid in withdrawal of fluid from the uterus.

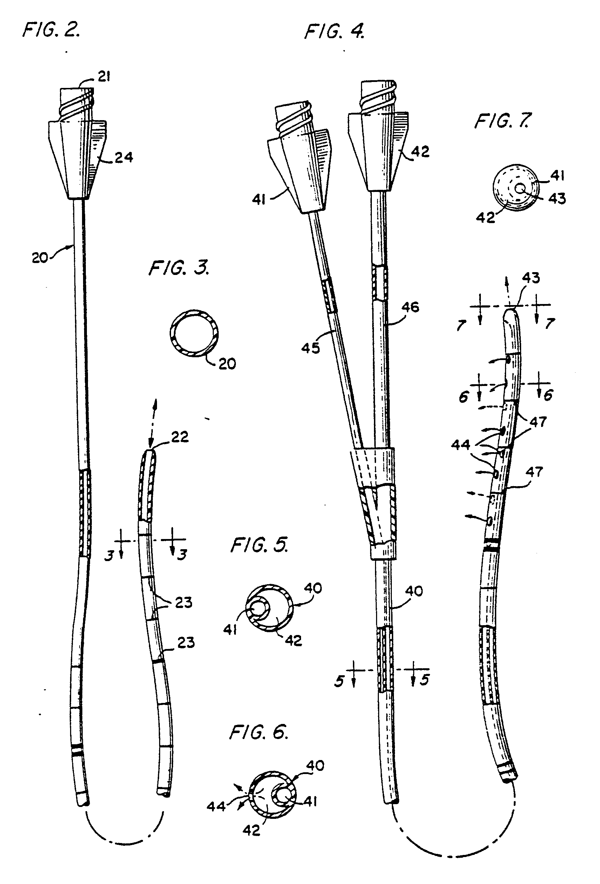

Figure 2 is an elevational view of a single lumen uterine catheter 20. Liquid is introduced into the adapter 24 at opening 21, flows through the uterine catheter 20, and empties into the uterus at opening 22. Markings 23 are placed along the length of the catheter 20 to aid in determining how far the catheter has entered into the uterus.

Figure 3 shows a transverse cross-sectional view of catheter 20.

Figure 4 is an elevational view of a double lumen catheter 40. Liquid is introduced through adapter 41 and flows out of lumen 45 through opening 43 in the frontal end of the catheter. Liquid flows back from the uterus into ports 44 in lumen 46 and out through adapter 42. Markings 47 are placed along the length of catheter 40 to aid in determining how far the catheter has entered into the uterus.

Figure 5 is an enlarged transverse cross-sectional view along line 5—5 of Figure 4 showing lumen 42 and lumen 41 of uterine catheter 40.

Figure 6 is an enlarged transverse cross-sectional view along line 6—6 of Figure 4 showing lumen 42 and lumen 41 and ports 44.

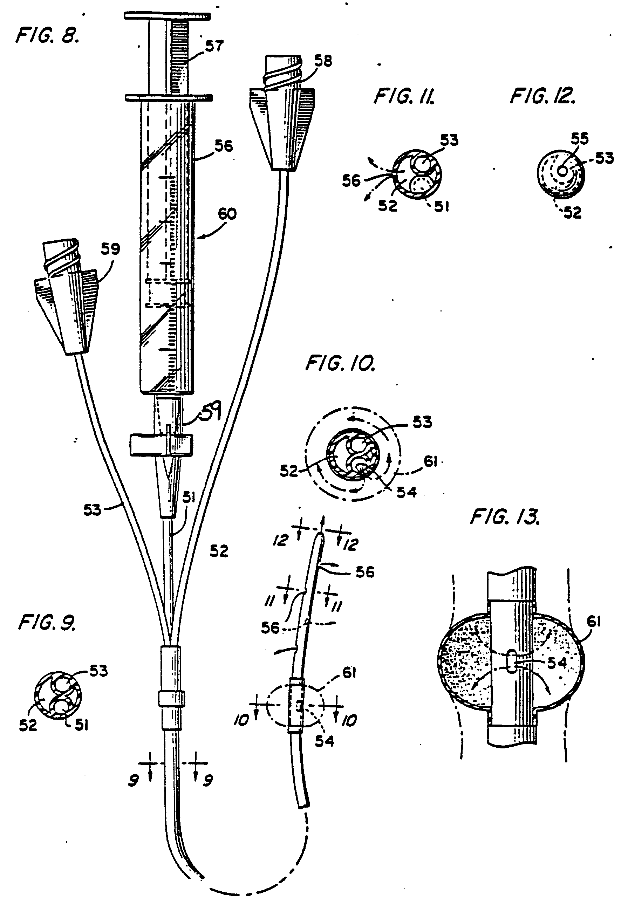

Figure 7 is an enlarged end view of uterine catheter 40 along line 7—7 of Figure 4 showing lumen 41 and lumen 42 and opening 43 at the tip of uterine catheter 40.

Figure 8 shows a triple lumen uterine catheter showing lumens 51, 52, and 53, and balloon (cuff) 61. Fluid is introduced at the proximal end into lumen 51 through adapter 59 by means of syringe 56. As plunger 57 is pushed down through the syringe barrel 60, fluid within the syringe barrel 60 will flow through lumen 51 out of port 54 and inflate the cuff 61. As plunger 57 is removed from syringe barrel 60, negative pressure will be created in cuff 61 and fluid will flow from the cuff 61 through port 54 into lumen 51 and deflate the cuff 61.

Fluid to be introduced into the uterus is introduced via the uterine catheter through adapter 58 through lumen 52 and out into the uterus through port 55 at the distal end. Fluid in the uterus is withdrawn through ports 56, through lumen 53, and out through adapter 59.

Figure 9 is an enlarged transverse cross-sectional view along line 9—9 of Figure 8 showing lumens 51, 52, and 53 of uterine catheter 50.

Figure 10 is an enlarged transverse cross-sectional view along line 10—10 of Figure 8 showing fluid flowing out of port 54 into cuff 61, as well as lumens 52, and 53.

Figure 11 is a transverse cross-sectional view along line 11—11 of Figure 8 showing lumen 51, lumen 52, and lumen 53, and port 56 in lumen 52.

Figure 12 is an end elevational view along line 12—12 of Figure 8 showing lumen 52 and lumen 53 with port 55.

Figure 13 is an enlarged fragmentary sectional view of the cuff portion of the uterine catheter 50. Cuff 61 is in inflated condition, the inflating fluid having entered the cuff 61 through port 54 in lumen 51.

As described above, the uterine catheter of the present invention is of flexible, non-rigid construction in order to follow undisturbed human contours and to allow conformation with various shapes, sizes, positions, and movements found in human cervical and uterine configurations. The uterine catheter possesses sufficient stiffness to permit entry by the device itself without need for a rigid stylet or stiffening rod and to prevent doubling or kinking of the tool. A ribbed construction of the wall of the catheter 20 may be made to achieve the necessary semi-flexibility. The catheter is of materials such as polyvinyl chloride, polyethylene, or Teflon® (polytetrafluoroethylene). The preferred catheter is transparent and may be desirably coated with Teflon® (polytetrafluoroethylene) or a silicone or similar slippery resin.

A method for evaluating the tubing for use in the uterine catheter of the present invention comprises measuring the deflection of the tubing, both vertically and horizontally, on a simple spring scale. A length of the tubing is held between the fingers, leaving a length of about 3 cm. exposed on one end. The free end is pressed down vertically on the scale. For suitability for use in the uterine catheter of the present invention, the tubing must possess the following characteristics:

1. The tube remains straight to a force of about 1—1/2 ounces (0.42 N).

2. The tube is bent to a "C" shape by a pressure of from about 4 ounces to about 8 ounces (1.11 to 2.22 N). The tubing remains straight and then suddenly gives way to said "C" curve.

The same tubing held in the same manner described above, is then applied to the scale horizontally or sideways with a 1/2 of cm. length on the scale. A force is exerted downwards onto the scale by the tubing, and the tubing remains approximately flat to a force ranging from about 2 ounces to about 6 ounces (0.56 to 1.67 N). The tubing then assumes a curve on the average of 45° deflection with a force of about one ounce to about 5 ounces (0.28 to 1.39 N). The tubing remains straight, then slowly gives way to a curve.

The uterine catheter may, in another embodiment, include an inflatable balloon cuff 61 for retention and blockage of flow of liquid introduced into the uterus. In this case, the uterus is not constantly irrigated, but the opening of the uterus is temporarily sealed with a small balloon, as shown in Figure 13, the uterus is infused with the flushing liquid, and the liquid is subsequently removed using a negative pressure.

The device is sufficiently soft to eliminate any chance of accidental perforation of the uterus. The preferred device also has, as will be noted in Figures 1 and 4, many apertures, a plurality of inlet apertures exceeding one, and a plurality of outlet ports exceeding two to enable the irrigation to be applied at a plurality of points and to permit recovery from a plurality of points along the body of the catheter.

A tool has now been described to facilitate non-surgical procedures for treating the uterus without causing discomfort to the patient. The tool, designated as a uterine catheter, is made of a soft, slippery material in order to move smoothly through the vaginal canal. However, the catheter has sufficient rigidity to allow it to penetrate the cervical os readily, as well as sufficient flexibility to enable it to conform to the cervical and uterine contours. The uterine catheter of the present invention is flexible to the extent that it can only be advanced stepwise about one or two centimeters at a time while held with blunt, smooth-tipped tweezers at the proximal end thereof. The uterine catheter of the present invention has a blunt, preferably bullet-shaped, nose to permit easy penetration of the cervical os or opening. The uterine catheter of the present invention has a small diameter to permit ready positioning of the catheter through the cervical os and into the uterus. The uterine catheter of the present invention has an exterior diameter of 1.6 millimeters to 4.0 millimeters, or about 5 French to 12 French. A two lumen device is preferably about 3 millimeters in outside diameter. The uterine catheter is generally from 150 to 200 millimeters in length.

The uterine catheter of the present invention can be used for a variety of non-surgical procedures for treating the uterus. For example, it can be used as a uterine probe, or as tool for flushing the uterus for embryo recovery or transfer, for flushing the uterus, testing for tubal patency, for introducing a treating material into the uterus, for opening the Fallopian tubes with fluid pressure, for endometrial sampling, for the recovery of ovum (or ova) (unfertilized eggs), for recovering and sampling uterine proteins and other contents, or for genetic engineering by injecting genetic material (including DNA) into the uterus.

For example, the uterus may be examined by penetrating and probing the uterus with the uterine catheter of the present invention. The uterus can be evaluated, using the uterine catheter, for ease of entry, depth, bumps, attachments, adhesions, such as scar tissue causing partial blockage, and the like. The uterine catheter may also be provided with a balloon, or several balloons of different sizes and different locations along the length of the catheter. The balloon(s) may be carefully and slowly inflated to enable the practitioner to evaluate the size and resiliency of the uterus.

The uterine catheter of the present invention is particularly useful in artificial embryonation, which will be described. The uterine catheter is used to introduce washing fluid into a uterus containing an embryo or ovum. The uterine catheter is then used to remove the washing fluid containing embryo or ovum from the uterus. A positive fluid pressure is used to introduce the washing fluid into the uterus, and a negative pressure is used to remove the washing fluid plus embryo or ovum from the uterus. The washing fluid containing ovum or embryo is then introduced into a second uterus, using positive pressure through the uterine catheter of the present invention.

In testing for tubal patency, a uterine catheter comprises one, two or three lumens and includes an inflatable balloon of from one to three cc. volume. The uterine catheter is slowly inserted into the uterus and the balloon is inflated and retracted, sealing the cervix against fluid leakage. A sterile fluid is slowly infused into the uterus over a period of about one to three minutes. Approximately 3 to 25 cc. of liquid are introduced into the uterus. The fluid that may be injected into the uterus may be a sterile salt solution such as saline; a solution of antibiotics or antimycotics, such as penicillin, streptomycin, griseofulvin; indicating dyes, or culture media. The disappearance of the fluid into the abdominal cavity indicates tubal patency. A gradual buildup of pressure, and the inability to infuse more than 3 to 8 cc. of fluid into the uterus, is an indication of blocked tubes.

The Fallopian tubes may be opened using the uterine catheter with inflatable balloon attached as described above, wherein the pressure is slowly increased to clear one or more blocked tubes by fluid flow.

The tubes may also be washed with treating solutions. The technique is as described above for introducing liquid into the tubes, using as the liquid a solution of a therapeutic material appropriate for the condition to be treated.

Some materials that may be introduced into the uterine tubes include radio-opaque dyes, heavy oils, and therapeutic materials such as antibiotics or antimycotics.

The uterine catheter can be used to change the DNA makeup of an embryo in utero, thus permitting desired traits to be introduced into the embryo. The embryo is removed from the uterus by flushing the embryo-containing uterus with the uterine catheter. Additional blastomers, from a different source or individual, are micro-inserted into the embryo, or new or different DNA or RNA material is micro-inserted into the embryo. The treated embryo is then re-introduced into a uterus using the uterine catheter of the present invention.

Uterine infections can be treated using the uterine catheter of the present invention by introducing treatment materials, such as antibiotics or antimycotics, into the uterus. The infusion of the material can be continuous, using the uterine catheter without balloons. Alternatively, a uterine catheter having an inflatable balloon attachment can be used to retain the fluid infused into the uterus for a required period of time for treatment.

The uterine catheter can be used to recover exfoliated endometrial cells from the uterus by introducing a liquid into the uterus and withdrawing the introduced liquid plus any exfoliated endometrial cells. The exfoliated cells are then available for further analysis.

In all of the above described procedures, the unique uterine catheter of the present invention can be inserted using the natural position of the cervix and the uterus with no need for repositioning of the cervix or the uterus. Because the natural position of the cervix and the uterus are used, there is no need to use a clamp or any other positioning tool. The procedures are painless to the patient due to the lack of necessity for repositioning the cervix or the uterus.

The uterine catheter may be made of a transparent material having the suitable flexibility/rigidity characteristics in order to permit observation of fluid contents and motion while the uterine catheter is in use.

Markings shown in Figure 2 are placed along the length of the catheter at predetermined intervals so that the uterine position may be exactly known and reproduced, particularly when the uterine catheter is used as a probe for the uterus.

Now, having described in detail my uterine catheter and described some of its methods of use, I will describe my preferred methods of embryo transfer.

Despite advances in embryo transfers in animals, advances from one species to another is speculative at best. Non-surgical recovery in cattle, for example, does not apply to humans. It is not possible to perform rectal palpation and manipulation of the uterus in humans. In the cow, the entire arm may be inserted in the rectum and the uterus lifted, positioned, and massaged.

Progress is based on biological analogies, but species by species invention is still required.

A different approach to embryo transfer in humans has been the use of external fertilization as an infertility treatment. The fertilization of a human egg in vitro appears first to have been accomplished in England in 1969 by physiologists R. G. Edwards and B. D Bavister, and gynecologist P. C. Steptoe. In the first recorded case, a human egg was surgically removed from a woman's ovary, fertilized externally, and then implanted in the same woman's uterus, where it developed to term. This in vitro fertilization/auto transfer (IVF/AT) specifically treats human infertility caused by blocked Fallopian tubes with a surgical method.

The present technique being disclosed is a treatment for infertility which does not require surgery, and may be used for the treatment of a wider range of causes of infertility. This process, also known as artificial embryonation, involves the artificial insemination of a fertile human female donor with semen from the husband of an infertile human female recipient. The resulting child does not carry the genes of the recipient, but does carry the genes of the husband and those of the donor. The recipient however, carries the fetus (for all but about five days) to birth.

Artificial embryonation thus gives the infertile woman the opportunity to nurture and give birth to a child. Many consider this a strong bonding experience which enables the recipient to fulfill the desire for the full measure of the experience of motherhood. Although the child does not carry the genes of the birth mother and is technically therefore not biologically related to her, it would be very difficult to convince the birthing mother that the child is not her own.

The differences between human non-surgical artificial embryonation and embryo transfers presently being conducted on animals are many.

In humans, the embryo donor must be fertile, but the recipient need not be fertile. The recipient need only possess a uterus which will support the growth and development of the embryo. This capability of the uterus is not affected by the recipient's non-production of eggs capable of being fertilized, as in the case of a recipient having blocked Fallopian tubes.

Embryo transplantation techniques presently used in cattle require painful manipulation of both donor and recipient animals necessitating the administration of general anesthetics. The procedure is a non-surgical, minimal trauma process which does not even involve dilation of the cervix or clamping of the cervix with a tenaculum. The procedure is carried out with natural cervical positioning rather than with arbitrary cervical repositioning and is therefore of minimal trauma, both to donor and recipient.

Embryo recovery by means of a non-surgical flushing procedure rather than a surgical procedure is painless and can be more efficacious. Such a non-surgical, minimum-trauma procedure can be performed more than once. It is possible, for example, to attempt to recover the fertilized embryo through flushing over a period of four days in a row, thus increasing the opportunity for a successful recovery.

Thus, the techniques described herein are believed to be especially useful for the treatment of human infertility, when the methods, devices and compositions described are employed as indicated.

The use of the processes described herein are to permit non-surgical embryo transplant, to avoid external fertilization, to treat certain forms of human infertility, and to recover various uterine and reproductive materials to enhance the study of reproduction and medical diagnoses.

Preferably, it will be preferred that donor's and recipient's menstrual cycles are synchronized to within plus or minus one day with respect to time of ovulation. A detailed knowledge of each menstrual cycle is obtained.

Synchronization is achieved by delaying onset of menses in the donor, recipient or both preferably by the administration of vaginal progesterone suppositories (50 mg. once or twice daily). This treatment is started at least three days before the cyclic drop in basal body temperature. Menses will begin 24—36 hours after progesterone withdrawal, and the ensuing cycle will generally be normal. Alternatively, any progestin, but typically progesterone $(C_{21}H_{30}O_2)$, is administered orally, topically, or parenterally, to either or both donor and recipient in order to assure that days of ovulation coincide. The support vehicle for progesterone varies depending upon the type of administration, and known vehicles may be used. Alternatively, synchronization can be achieved by administration of an effective amount of a compound selected from the group of progestins, estrogens (especially estradiol-17 beta), human chorionic gonadotrophin, clomiphene citrate, follicle stimulating hormone, and mixtures thereof in a suitable support vehicle.

After appropriate synchronization has been achieved, the embryo donor is artificially inseminated with semen from the husband of the infertile recipient. A deep cervical insemination technique is used.

After fertilization, the fertilized egg moves along the oviduct, dividing as it goes. The egg usually reaches the uterus about three days later as a morula, a spherical cluster of eight or more cells. The division of the cells continues in the uterus to produce a blastocyst — a liquid-filled sphere with a group of cells, the inner cell mass, at one side. The blastocyst is surrounded by a clear, thick, gelatinous coating of proteinaceous material called the zona pellicida. The blastocyst 'floats' freely in the uterus for several days. At approximately 7—8 days, the 100—1000 cell embryo "hatches" from the zona pellicida, and only then is the hatched blastocyst embryo capable of implantation, which presumably occurs within 48 hours after hatching.

Non-surgical embryo recovery involves the non-surgical removal of the fertilized egg (through flushing) from the donor's uterus, prior to implantation. Since the egg does not implant until approximately three days after entry into the uterus, the non-surgical flush can recover the embryo which can then be transferred to the recipient.

A double lumen cannula tool, which I have described, is used to introduce washing fluid into the uterus as well as to remove the washing fluid and ova or embryo(s) from the uterus. A positive pressure is used to introduce the washing fluid into the uterus, and a negative pressure is used to remove the washing fluid from the uterus. The preferred flushing tool for recovery of ova or embryos has a smooth rounded tip to prevent catching of the uterus, and is preferably between 1.8 mm and 3.5 mm in external diameter. The flushing tool is of flexible, non-rigid construction in order to follow undistorted human contours and to allow conformation with various shapes, sizes, position and movements found in human cervical and uterine configurations. The flushing tool possesses sufficient stiffness to permit entry by the device itself without need for a rigid stylet or stiffening rod and to prevent doubling or kinking the tool. A ribbed construction, which preferably is external of the catheter 20, can be used to prevent clogging. The flushing tool may be of materials such as polyvinyl chloride, polyethylene or Teflon® (polytetrafluoroethylene), or may be coated with Teflon® (polytetrafluoroethylene) or silicone.

The flushing tool has sufficient stiffness that a 4 cm length will resist, without bending, a vertically applied weight of up to 15 grams; and a 4 cm length will bend under the application of a horizontally applied weight of 10 grams at a distance of 1.0 to 3.0 cm. The exterior surface of the tool is slippery, with a coefficient of friction between 0.03 and 0.15. Such a tool penetrates the human uterus and yet will conform to the various irregularities, shapes and sizes characterizing a given human female reproductive system.

The flushing tool may include an inflatable cuff for retention and blockage of flow of the flushing fluid. In use, the tool does not irrigate the uterus constantly. The uterus is temporarily sealed with a small balloon, inflated with the flushing fluid, and the liquid is subsequently removed using a negative pressure.

The flushing involves the use of the medium described herein. Preferably an intravenous stand is employed. A flushing fluid container is placed on the stand, a tube connected between the catheter and the container, and the height of the container adjusted up or down to obtain the desired fluid column pressure. Each meter that the container is raised above the inlet provides approximately 1/15th of atmospheric pressure to the column of fluid. The outlet may also be used to vary pressure by the use of a syringe to give positive or negative pressure to the outlet fluid. A combination of these procedures is normally and preferably used.

The flushing fluid consists of tissue culture liquid raised to body temperature. The fertilized egg or embryo is washed out in the fluid through one channel of the flushing tool.

The fluid used may be one of several suitable for tissue culture. Examples include Dulbecco's phosphate buffered saline, PBS, as described in the *Journal of Experimental Medicine*, 98:167 (1954) as an aqueous solution of calcium chloride, potassium chloride, potassium dihydrogen phosphate, magnesium chloride, sodium chloride, and sodium hydrogen phosphate; and Gibco Medium 199 or TCM-199, as described in *Proceedings of the Society of Experimental Biology and Medicine*, 73:1 (1950) as an aqueous solution of calcium chloride, ferric nitrate, potassium chloride, potassium dihydrogen phosphate, magnesium sulfate, sodium chloride, sodium bicarbonate, and sodium hydrogen phosphate. These media should be buffered to pH 7.0 to 7.5 and preferably in the range of pH 7.3 to 7.4.

Additives to the basic tissue medium may include human serum albumen (up to 20 percent) or human uterine protein (as flushed and filtered; between 0.02 and 10.0 percent). With other mammals, an analogous serum is preferred.

Alternatively, the flushing process can be used to harvest human embryos or ova from the uterus, singly or in multiples following superovulation. The embryos or ova can be frozen for storage, and may then later be thawed and cultured for auto-transfer (into the woman from whom they came).

After the embryo is flushed from the uterus of the donor, the liquid culture media containing the embryo is examined under a microscope and the egg is located. Unlike cow or mouse embryos, for which the greatest numbers of transplants have been done, the human embryo is very difficult to find, since it is nearly transparent. After the embryo is located, it is transferred to fresh media and carefully examined under a high resolution microscope for fertilization, division, cell structure and organization. A preferred embryonic developmental stage for transfer is the 50—100 cell stage.

Under ideal transfer conditions, the embryo may be maintained externally for a period as short as 20 to 30 minutes. If it is maintained for a longer period, the culture medium may be enhanced by modification of energy sources. As requirements of the embryo change, the culture may be modified to include glucose, fructose, pyruvate, citric acid, alpha-keto-glutorate, or ATP. Co-culturing the embryo with endometrial tissue or individual endometrial cells enhances the process.

The externally maintained embryo is held in an incubator in which the atmospheric environment typically contains 0.5 to 5 percent $CO_2$, up to 95 percent relative humidity, 5 to 15 percent oxygen, balanced nitrogen, and controlled temperature and temperature distribution, typically 37°, to avoid temperature shocks on entry and removal.

Alternatively, embryos may be frozen and stored for later use.

Problems in freezing and storage of embryos include the mechanical and biochemical destruction of cells by the expansion of water upon freezing. Therefore, it is essential to use cryoprotective agents which perform several functions: 1) reduce the amount of water in the cell without killing it, 2) reduce the expansion upon freezing, 3) develop uniformly distributed micro-

crystals instead of a few large macro-crystals, and 4) are not poisonous in and of themselves.

Freezing is done slowly to permit cellular accommodation. A final solidification often does not occur until −60 or 80°C, with many cryo-protective agents and cellular constituents.

These conditions can be satisfied in whole or in part by using stepwise serial concentrations of cryo-protective agents prior to freezing and in conjunction with temperature lowering, and inversely, using stepwise serial dilution of cryo-protective agents upon thawing and warming. Cryo-protective agents include DMSO (dimethyl sulfoxide), and glycerol.

The embryo is non-surgically transferred to the recipient by a flushing technique similar to that used to flush the embryo from the donor. A second small instrument is inserted through the vagina and through the cervix into the body of the donor's uterus. The embryo is then injected into the uterus, with a small amount of culture fluid.

Some of the hormonal processes in oocyte maturation, ovulation, fertilization, in the ampulla, oviductal transport, uterine, 'float', and embryo hatching should be here noted.

A detailed understanding of the interrelated hormonal processes that control the female reproductive cycle has been of particular importance in developing embryonic transplants and external fertilization techniques. The major anatomical elements involved in the cycle are the hypothalmus near the base of the brain, the pituitary gland just below it, the ovaries, the oviduct, the uterus and the vagina. In each turn of the normal human female reproductive cycle, one egg matures, is discharged from the ovary, and passes into the expanded end of the oviduct, down which it moves to the uterus. In the absence of fertilization, the egg or ova disintegrates. Without implantation, the cycle is repeated at monthly intervals. If mating or insemination has occurred at the right time in the cycle, the egg may meet sperm in the oviduct at the ampulla and may become fertilized.

The fertilized egg, or embryo, then moves slowly along the oviduct, nurtured and dividing as it goes. The egg usually reaches the uterus about three days later as a morula—a spherical cluster of 12 or more cells. The division of the cells continues in the uterus to evolve into a blastocyst, a fluid-filled spherical body with a group of cells, the inner mass at one side. The hatched blastocyst adheres to the endometrium, the lining of the uterus—and a complex implantation process embeds the blastocyst in the uterine wall, where development continues. The inner cell mass becomes the fetus; the outer cells of the blastocyst, together with neighboring uterine cells and blood vessels, form the placenta, the organ that provides for the exchange of materials between the later embryonic circulation and the maternal circulation.

These events are regulated by the interplay of hormones produced in the hypothalmus, the pituitary, the ovaries, and the placenta. Follicle-stimulating hormone (FSH) is secreted by the pituitary and stimulates growth of the ovum in the ovary. As the ovum grows, its cells secrete estrogenic hormones, chiefly estradiol, which acts on the pituitary to reduce FSH while simultaneously stimulating growth of the uterine wall and its glands.

As the follicle approaches full size, FSH output decreases and a second pituitary hormone, luteinizing hormone (LH), becomes predominant. LH completes the maturation of the egg and triggers its discharge into the oviduct. Residual follicle cells left after the discharge of the ovum secrete progesterone, which induces further uterine wall development. If fertilization and pregnancy do not occur, a new set of ovarian follicles grows and the corpus luteum derived from the ovulated follicle begins to decline. The resulting change in the estradiol/progesterone balance precipitates degenerative changes in the uterine wall, and menstruation occurs.

If the egg has been fertilized, however, the outer cells of the deloping embryo or blastocyst secrete HCG (human chorionic gonadotrophin), a hormone which functions must like LH. HCG acts to prevent the development of new follicles in the ovary. After approximately five weeks, the placenta takes over, secreting progesterone to maintain pregnancy.

Superovulation can be achieved by the administration of FSH which causes the maturation of several follicles rather than a single one. The increased frequency of human quintuplets and even sextuplets in recent years reflects the frequent administration of FSH in treating infertility due to reduced egg production.

The technique can also increase the availability of eggs for in vitro fertilization or embryonic transplant. The eggs have traditionally been recovered from the ovary by laparoscopy, a surgically invasive procedure developed for diagnostic purposes. A tiny telescope and a fiber-optical illuminator are introduced through a small incision in the region of the navel; the ovary and any mature follicles can be examined, and eggs can be removed, generally by aspiration, from the follicles.

External maintenance of the egg presents a problem. The egg is normally suspended in the complex biological fluids that are present in the oviduct and the uterus. Initially, there was little information about which constituents of these fluids were essential for continued health of the egg.

A similar problem had to be faced with the sperm, the other component of normal fertilization. The sperm are obtained in large numbers from ejaculated semen. An important obstacle had to be dealt with, however, before external human fertilization could be accomplished, whether for artificial insemination of a donor female or for insemination in vitro.

In the initial ejaculate, the sperm are not prepared to penetrate the egg. They must first undergo capacitation, a process that normally is induced by conditions in the female genital tract. These conditions must be simulated externally if the sperm are to be in the proper state to penetrate the egg.

One of the problems associated with in vitro fertilization, then, is to prove that a sperm enters an undamaged egg and that both the egg and the sperm then function normally. Although there are intermediate steps in this validation, the final test is the normal development of the zygote, or fertilized egg. In the case of eggs that normally develop externally, for example, those of starfish in seawater or of frogs in pond water, this is no great obstacle. However, for eggs that normally develop internally, the problem becomes difficult. Two options are available: return of the egg to its normal internal environment, or the development of a suitable external environment. As a therapeutic procedure in human beings there is no question that the first option is biologically simpler and currently far more socially acceptable.

Given the close hormonal control of the normal events in ovulation, fertilization, oviductal transport, uterine maintenance, and implantation of the egg, however it is clear that if implantation of an externally fertilized zygote is to be successful, the state of the zygote must be closely matched to the state of the uterine wall. If, for example, an egg that is fertilized after removal from a follicle develops more slowly outside the oviduct than within it, the egg might be introduced into the uterus of the recipient in too premature a state to stimulate an implantation reaction, or it might be introduced at a point in time which is too late because the uterus is already past its optimal time to permit implantation.

A non-surgical non-anesthetic, painless office procedure for transplanting a fertilized egg from a fertile human female donor to an infertile human female recipient is one result of our described method. This process may also be referred to as artificial embryonation, a term coined and defined by the inventor to emphasize that artificial embryonation is analogous to artificial insemination. The fertile donor may be artificially inseminated with semen from the husband of the infertile recipient. The resulting child does not carry any of the genes of the recipient, but does carry the genes of the husband and those of the donor. The recipient carries the fetus (for all but about five days) to birth.

Processes for artificial embryonation and tissue recovery may advantageously be carried out using the uterine catheter of this invention.

Generally, the process of artifical embryonation is used when the donor is a fertile female and the recipient is an infertile female. Alternatively, the process of artificial embryonation can be used when an infertile couple adopts an embryo which is transplanted into the infertile wife's uterus. In this case, the embryo carries the genes of neither the wife nor the husband, and the process does not require fertility on the part of either husband or wife.

The process of artificial embryonation is also useful in the instance of a woman not capable of carrying an embryo to term. A second woman can be used as a substitute uterine carrier to carry the embryo to term for the donor of the ovum.

The process of artificial embryonation can also be used in the case wherein a donor produces a viable embryo but cannot carry it to term. In this case, the embryo can be transferred to a substitute uterine carrier, who carries the embryo to term. This process is thus useful for the case of a donor with an incompetent cervix, or of a donor who because of disease or paralysis, is advised that a pregnancy would be dangerous.

The uterine catheter of the present invention can be used to harvest embryos which may be frozen for storage, amd may then later be thawed, and cultured for auto-transfer (into the woman from whom they came) or for hetero-transfer (into a woman other than the one from whom they came). The oocytes are preferably frozen and stored in liquid nitrogen.

In order for the egg of a donor to be successfully transplanted into a recipient, the ovulation of both the donor and the recipient must be synchronized. Any progestin, typically progesterone, may be administered vaginally, intra-uterine, orally topically, or parenterally, to one or both of the females to delay the onset of menstruation so that the ensuing cycle days of ovulation coincide.

The approximate length of the menstrual cycle and the approximate day of ovulation within the cycle must be known for each individual. Typically, the onset of menstruation of the donor is delayed hormonally; sequential cycles may then be synchronized by delay of the recipient or further delay of the donor.

Once ovulation of both the donor and the recipient has been synchronized, the fertile donor is artificially inseminated. The uterus of the donor is then non-surgically flushed to remove the resulting oocyte (fertilized egg), and the oocyte is isolated and cultured. The oocyte is then non-surgically transvaginally and transcervically transferred to the uterus of the recipient.

The differences between human non-surgical artificial embryonation and the embryo transfers presently being conducted on animals are many.

In the case of humans, the egg donor must be fertile, but the recipient need not be fertile—she need only possess a uterus that will support the growth and development of the embryo to term. Obviously, this capability of the uterus is not necessarily adversely affected by the lack of production of eggs capable of being fertilized, as in the case of blocked Fallopian tubes.

The embryo transplantation techniques presently used in cattle require painful manipulation of both the donor and recipient animals and necessitate the administration of a local or general anesthetic. Artificial embryonation using the uterine catheter of the present invention is absolutely painless, and does not even involve dilation of the cervix or clamping of the cervix with a tenaculum. Artificial embryonation using the uterine catheter of the present invention is carried out with natural cervical positioning rather than with arbitrary cervical repositioning and therefore is a painless procedure to both the donor and the recipient.

Multiple flushes of the donor's uterus to recover the ovum are possible, because the procedure is painless and the donor is not reluctant to undergo the flushing procedure more than once. This permits flushing, for example, for four days in a row, in order to ensure recovery of a visible ovum.

Preferably, a double lumen cannula tool is used to introduce washing fluid into the uterus as well as to remove the washing fluid and ova or embryos from the uterus. A positive pressure is used to introduce the washing fluid into the uterus, and a negative pressure is used to remove the washing fluid from the uterus. The preferred flushing tool, which we described, for recovery of ova or embryos has a smooth rounded tip to prevent catching of the uterus, is preferably between about 1.8 mm and about 3.5 mm in external diameter, and is of flexible, non-rigid construction in order to follow undistorted human contours and to allow conformation with various shapes, sizes, positions and movements found in human cervical and uterine configurations. The flushing tool possesses sufficient stiffness to permit·entry by the device itself without need for a rigid stylet or stiffening rod and to prevent doubling or kinking of the tool. A ribbed construction on the exterior of the catheter can be used to prevent clogging.

Alternatively, the flushing tool includes an inflatable cuff for retention and blockage of flow of the flushing fluid. When such a tool is used, the uterus is not constantly irrigated, but the opening of the uterus is temporarily sealed with a small balloon, the uterus is inflated with the flushing fluid, and the liquid is subsequently removed using a negative pressure.

In summary, artificial embryonation is a non-surgical, non-anesthetic, transcervical, transvaginal office procedure for both donor and recipient.

Embryo transplant is possible because of two fundamental biological phenomena:

1. There is no immune response to an embryo. Any female can carry an embryo from the same species.

2. The human embryo floats relatively freely in the uterus from approximately about the third day to the seventh day of pregnancy. The embryo does not begin to attach and bury itself in the uterine wall until about the eighth through the tenth day of pregnancy. Thus, the embryo can be washed or flushed out during the approximately four-day "window" period. The "window" period varies between species. For example, the comparable "window" period for cattle is an eleven-day period from the fourth to the fifteenth day of pregnancy.

A critical step in the embryonic transfer is the physical transfer of the fertilized egg from the donor to the recipient. A slender, curved, perforated instrument is inserted through the vagina into the uterus. A specially formulated tissue culture liquid is raised to body temperature and flushed through the flushing tool into the uterus. The oocyte or embryo is washed out in the fluid through another channel in the flushing tool. In the process, the uterus of the donor is flushed at about four to seven days following artificial embryonation.

The liquid culture media containing the oocyte is then examined under a microscope and the oocyte is located. The oocyte is transferred to fresh media in a special dish and carefully examined under a high quality microscope for fertilization, cell division, cell structure, and organization. The ideal embryo is in the 50—100 cell stage.

The embryo is then transferred to the recipient in a manner similar to the first flushing. A second small instrument is inserted through the vagina and through the cervix into the body of the recipient's uterus. The embryo is injected into the uterus with a small amount of special tissue culture fluid. The menstrual cycles of the donor and the recipient have previously been synchronized by the use of progesterone and/or other hormones to insure similar uterine and hormonal environments for the transferred embryo.

For the brief period duing which the embryo is outside a host body, every effort is made to maintain it in an environment as similar to the body as possible. The environment for the embryo is controlled by the use of special incubators and special media. In a normal transfer, the embryo would be outside the body for between 20 and 30 minutes. The humidity, acidity and vapor pressure of carbon dioxide, and nitrogen are all carefully controlled. Alternatively, the embryo may be preserved in liquid nitrogen until it is transplanted into a recipient.

The overall procedure for both donor and recipient is no more uncomfortable or hazardous than the fitting of an intra-uterine device. No surgery is involved for either the donor or the recipient.

Alternatively, an oocyte may be taken from a woman who chooses to bear children at some later date, and stored in liquid nitrogen, in much the same way as sperm is stored in sperm banks. This "oocyte bank" may be of particular use to women who wish to postpone having children for professional reasons, or for women who, for reasons of convenience, prefer to save oocytes in the event that they change their minds after having undergone a sterilization procedure.

The method used for recovery of ova may be used for preservation of the fertility of the subject which is useful, for example, when the subject desires to have the tubes ligated. The procedures for such recovery are the same as previously described, except that the oocyte or embryo (if the oocyte was fertilized) can be stored as in sperm banks by freeezing also as previously described.

The process which has been developed for use in ova recovery is exceptionally painless and does not involve surgery. This process allows for preventing unwanted pergnancies in humans.

The process for recovery is the same process as that employed to recover ova from the uterus. The uterine catheter is stepwise inserted into the uterus, transvaginally and transcervically. The balloon is inflated. Fluid is introduced and the ova flushed and recovered before implantation and discarded.

This process is used on the third or fourth day after copulation, when an unwanted pregnancy could likely occur, and may be repeated one or more of three successive days.

Here I prefer to use an effective amount of ova destructive agents in the flushing medium selected from the group consisting of estrogens (estradiol-17 beta), hyperosmotic compounds, glucose, prostaglandins, acids, alkalis and urea, and mixtures thereof. These agents are normally mixed with normal saline for flushing.

Embryo transplants in humans involve difficulties, risks, and problems not present or applicable in animals. For example:

a) Failure to recover the embryo results in an unwanted pregnancy in the donor. Repeat flushing and embryocidal agents described herein control this problem.

b) A reverse flush (into the oviduct) can create accidental tubal pregnancy. We have developed inlet and outlet pressure control techniques to eliminate this problem. This is accomplished by using a standard intravenous stand with a media contained in a fluid controller, which has tubes leading to the catheter. Adjustment of the height of the container relative to the subject controls the pressure of the fluid entering the catheter. As we have said, one meter would provide an additional 1/15th of atmospheric pressure to the column of fluid. The negative pressure or suction controls the outlet as previously described. We utilize pregnancy monitoring techniques in the donor to assure embryo recovery and avoid non-uterine pregnancy.

c) Twin ovulation occurs about 1 out of 80 times, and the repeat flushes with embryocidal agents and pregnancy monitoring control this problem.

d) Naturally occurring ectopic pregnancy in the donor must be carefully monitored as described above.

e) Particular attention must be paid to obtaining informed consent of donor and recipient for these procedures. Any procedure performed on humans requires elaborate care, precaution, and expertise that can only come from the performance of thousands of animal experiments with subjects closely approximating those of the human.

I should here also state that most of the methods disclosed herein are applicable to the human species. Species to species comparisons are not generally effective, as I have said. In particular, we have learned from experience that rodents, who do not have the cycles we are dealing with, are not good teaching tools.

Similarly, the deomesticated farm animal is not a good analog for much of my work. These animals react differently to the compounds used, and although not permitted by human anatomy manipulation of the reproductive tract through the rectal wall is possible particularly in cattle and horses.

Nevertheless, my tools will be particularly useful in the smaller animals and those that would otherwise require surgery particularly primates. The use of my methods and apparatus with primates particularly and with other mammals which do not allow for palpation through the rectum is contemplated and I believe will substantially advance the treatment of these animals as well as permit non-surgical transfers.

**Claims**

1. A uterine catheter (20 or 40) permitting introduction of fluid into a human uterus without surgery or rectal palpation and having at least one lumen and an open forward distal end adapted for insertion into a human uterus and an open rear proximal end (21) adapted to permit connection to a source of fluid, the distal end of said catheter having a rounded tip (22) characterized in that said catheter has sufficient rigidity to permit insertion into the uterus by itself, without the need for repositioning of the uterus and has sufficient flexibility to permit conformation to human cervical and uterine configurations.

2. The catheter of claim 1, which comprises:

a) a semi-flexible tubular catheter (40) having fluid passage (45, 46) means axially extending therethrough, said catheter having a distal end and a proximal end, and said fluid passage means extending from said proximal end to said distal end,

b) fluid coupling means (41) located at the proximal end of said catheter for coupling one of said fluid passage means to an external supply of fluid, and,

c) aperture means (42, 44) adjacent to the distal end of the catheter in communication with said fluid passage means (45) for entry of liquid into the uterus and for recovery of liquid from the uterus.

3. The catheter of claim 1, having at least one proximal aperture and one interconnected distal aperture adapted for the entry of fluid from said proximal end to said distal end and for a recovery of said fluid from the area of the body adjacent to the distal end.

4. The catheter of claim 1, having two lumens (40).

5. The catheter of claim 1, having three lumens (50).

6. The catheter of claim 1, having means to seal off the opening of the uterus when the catheter is in place in the uterus.

7. The catheter of claim 6, wherein the means to seal off the opening of the uterus is an inflatable ballon cuff (61).

8. The catheter of claim 1, wherein a plurality of distal apertures (44) are arranged in a spiral configuration along the end portion of the catheter.

9. The catheter of claim 1, made of a transparent material.

10. The catheter of claim 1, made of a material which is polyvinylchloride, polyethyene or polytetrafluorethylene.

11. The catheter of claim 1, wherein markings are placed at regular intervals on the outside of the catheter.

12. The catheter of claim 1, having sufficient flexibility and rigidity to permit said catheter, when gripped and leaving a three centimeter length as a free end which is vertically pressed downward on a scale, to remain straight to a force of about one and one-half ounces 0,42 N) and to be bent to a "C" shape by a pressure of from about four to eight ounces (1.11 to 2.22 N).

13. The catheter of claim 1, having a flexibility and a rigidity which is measurable by gripping a section thereof leaving three centimeters as a free end and which after gripping and being pressed downwardly on a scale with approximately one half centimeter oriented horizontally on the scale to read in the range between about one ounce and five ounces (0.28 to 1.39 N) when said free end of said section is bent to a curved shape at approximately 45° deflection.

14. The catheter of claim 1, having a flexibility and rigidity such that a three centimeter length of the catheter when gripped leaving three centimeters as a free end has been pressed vertically downwardly on a scale will remain straight up to a downward force of about one and one half ounces (0.42 N) and the catheter will bend thereafter with continued additional downward force.

15. The catheter of claim 1, having a flexibility and rigidity such that three centimeters of length of the catheter when gripped leaving three centimeters as a free end has been vertically pressed downwardly on a scale with the free end pressed against said scale will remain straight and with original shape and then will bend at a downward pressure ranging between about four and eight ounces (1.11 to 2.22 N).

16. The catheter of claim 1, having an external diameter between about 5 and 12 French.

17. The catheter of claim 1, having a length between about 150 and 200 millimeters.

18. The catheter of claim 1, which is a triple lumen catheter (50) having a balloon cuff (61) positioned at a spaced distance from the distal end of the catheter wherein said apertures are forward of said balloon, said catheter being provided with balloon filling means enabling the balloon to be filled and expanded in order to block liquid passage from the reproductive tract other than through an aperture in the distal end of the catheter.

19. The catheter of claim 1, which is a triple-lumen catheter having second fluid passage means and second aperture means in communication with said second fluid passage means such that said catheter permits entry of liquid into the uterus via one of said fluid passage means and exit from the uterus via the other of said fluid passage means.

20. The catheter of claim 1, having a variety of inlet apertures and outlet apertures to permit introduction of fluids into and recovery of fluid from the uterus.

**Patentansprüche**

1. Uteruskatheter (20 oder 40), der das Einführen eines Fluides in einen menschlichen Uterus ohne chirurgische Eingriffe oder rektales Tasten erlaubt und mindestens ein Lumen sowie ein offenes vorderes zum Einführen in einen menschlichen Uterus ausgebildetes distales Ende und ein offenes rückwärtiges mit einer Fluidquelle verbindbares proximales Ende aufweist, wobei das distale Ende des Katheters eine abgerundete Spitze (22) aufweist, dadurch gekennzeichnet, daß der Katheter eine hinreichende Steifigkeit aufweist, um sein eigenständiges Einführen in den Uterus ohne die Notwendigkeit einer Ausrichtung desselben zu erlauben, und eine hinreichende Flexibilität besitzt, um seine Anpassung an menschliche Gebärmutterhals- und Uterusformen zu ermöglichen.

2. Katheter nach Anspruch 1, umfassend:
a) einen habflexiblen schlauchförmigen Katheter (40) mit sich axial durch diesen erstreckenden Fluiddurchtrittsmitteln (45, 46), wobei dieser Katheter ein distales und ein proximales Ende aufweist und sich die Fluiddurchtrittsmittel vom proximalen zum distalen Ende erstrecken,
b) an dem proximalen Ende des Katheters gelegene Fluidkupplungsmittel (44) zum Koppeln eines der Fluiddurchtrittsmittel mit einer externen Fluidquelle und
c) dem distalen Ende des Katheters benachbarte und mit den Fluiddurchtrittsmitteln (45) in Verbindung stehende Öffnungsmittel (43, 44) zum Eintritt von Flüssigkeit in den Uterus und zur Wiederaufnahme von Flüssigkeit aus dem Uterus.

3. Katheter nach Anspruch 1 mit mindestens einer proximalen Öffnung und einer mit dieser verbundenen distalen Öffnung zum Eintritt von Fluid von dem proximalen Ende zu dem distalen Ende und zur Wiederaufnahme des Fluides von dem dem distalen Ende benachbarten Körperbereich.

4. Katheter nach Anspruch 1 mit zwei Lumen (40).

5. Katheter nach Anspruch 1 mit drei Lumen (50).

6. Katheter nach Anspruch 1 mit Mitteln zum Abdichten der Öffnung des Uterus, wenn der Katheter sich in dem Uterus befindet.

7. Katheter nach Anspruch 6, wobei die Mittel zum Abdichten der Öffnung des Uterus von einer aufblasbaren Ballonmanschette (61) gebildet sind.

8. Katheter nach Anspruch 1, wobei eine Mehrzahl von distalen Öffnungen (44) in spiraliger Konfiguration entlang dem Endabschnitt des Katheters angeordnet sind.

9. Katheter nach Anspruch 1, hergestellt aus einem transparenten Material.

10. Katheter nach Anspruch 1, hergestellt aus einem Material, das Polyvinylchlorid, Polyäthylen oder Polytetrafluoroäthylen ist.

11. Katheter nach Anspruch 1, wobei an der Außenseite des Katheters Markierungen in regelmäßigen Abständen angeordnet sind.

12. Katheter nach Anspruch 1 mit einer hinreichenden Flexibilität und Steifigkeit, damit der Katheter, wenn er erfaßt und ein 3 cm langes Stück als freies Ende gelassen wird, das vertikal abwärts auf eine Waage gepreßt wird, bis zu einer Kraft von etwa 1 1/2 Unzen (0,42 N) gerade bleibt und zu einer C-Form gebogen wird bei einem Druck von etwa 4 bis 8 Unzen (1,11 bis 2,22 N):

13. Katheter nach Anspruch 1 mit einer Flexibilität und Steifigkeit, die meßbar ist, indem man einen Abschnitt desselben unter Freilassen von 3 cm als freies Ende erfaßt und die nach dem Erfassen und Andrücken auf eine Waage unter horizontaler Ausrichtung von annähernd einem halben cm auf der Waage einen Wert im Bereich von ungefähr einer und fünf Unzen (0,28 bis 1,39 N) ergibt, wenn das freie Ende des Abschnitts mit annähernd 45° Auslenkung zu einer gekrümmten Form gebogen wird.

14. Katheter nach Anspruch 1 mit einer Flexibilität und Steifigkeit derart, daß ein 3 cm langes Stück des Katheters, wenn dieser unter Freilassen eines 3 cm langen Endes erfaßt und vertikal abwärts auf eine Waage gedrückt wird, gerade bleibt bis zu einer abwärts gerichteten Kraft von ungefähr 1 1/2 Unzen (0,42 N) und daß der Katheter sich anschließend bei zunehmender abwärts gerichteter Kraft biegt.

15. Katheter nach Anspruch 1 mit einer Flexibilität und Steifigkeit derart, daß ein 3 cm langes Stück des Katheters, wenn dieser unter Freilassen eines 3 cm langen Endes erfaßt und vertikal abwärts auf eine Waage gedrückt wird, gerade und in seiner ursprünglichen Form bleibt und sich anschließend biegt bei einem abwärts gerichteten Druck im Bereich von ungefähr 4 bis 8 Unzen (1,11 bis 2,22 N).

16. Katheter nach Anspruch 1 mit einem Außendurchmesser von ungefähr 5 bis 12 French.

17. Katheter nach Anspruch 1 mit einer Länge zwischen 150 und 200 Millimetern.

18. Katheter nach Anspruch 1, der ein Drei-Lumen-Katheter (50) ist mit einer Ballonmanschette (61), die in einem Abstand vom distalen Katheterende angeordnet ist, wobei die genannten Öffnungen vor dem Ballon liegen und wobei der Katheter mit Ballonfüllmitteln versehen ist, die ein Füllen und Expandieren des Ballons ermöglichen, un einen Flüssigkeitsdurchtritt aus dem Fortpflanzungstrakt nur durch eine Öffnung in dem distalen Katheterende zuzulassen.

19. Katheter nach Anspruch 1, der ein Drei-Lumen-Katheter ist mit zweiten Fluiddurchtrittsmitteln und zweiten Öffnungsmitteln, die mit den Fluiddurchtrittsmitteln derart verbunden sind, daß der Katheter den Eintritt von Flüssigkeit in den Uterus über eines der Fluiddurchtrittsmittel und den Austritt über das andere der Fluiddurchtrittsmittel erlaubt.

20. Katheter nach Anspruch 1 mit einer Vielfalt von Einlaßöffnungen und Auslaßöffnungen, um das Einführen von Fluiden in den und die Wiedergewinnung von Fluid aus dem Uterus zu ermöglichen.

**Revendications**

1. Cathéter utérin (20, 40) permettant l'introduction de fluides dans un utérus humain sans chirurgie ni palpation rectale et ayant au moins un passage et une extrémité distale avant ouverte adaptée à son insertion dans un utérus humain et une extrémité proximale arrière ouverte (21) adaptée pour permettre son raccordement à une source de fluide, l'extrémité distale de ce cathéter ayant un bout arrondi (22), caractérisé en ce qu'il a une rigidité suffisante pour permettre son insertion par lui-même dans l'utérus, sans la nécessité du repositionnement de l'utérus, et il a une flexibilité suffisante pour lui permettre de se conformer aux configurations cervicale et utérine humaines.

2. Cathéter de la revendication 1 qui comprend:

a) un cathéter tubulaire (40) semi-flexible ayant des passages (45, 46) de fluide s'étandant en sens axial sur sa longueur, ce cathéter ayant une extrémité distale et une extrémité proximale et lesdits passages de fluide s'étendant de ladite extrémité proximale à ladite extrémité distale,

b) un moyen (41) de raccordement pour fluide situé à l'extrémité proximale dudit cathéter pour le raccordement de l'un desdits passages de fluide à une source extérieure de fluide, et,

c) des ouvertures (43, 44) adjacentes de l'extrémité distale du cathéter en communication avec lesdits passages de fluide (45) pour l'entrée de liquide dans l'utérus et pour la récupération de liquide dans l'utérus.

3. Cathéter de la revendication 1 ayant au moins une ouverture proximale et une ouverture distale interconnectées adaptées à l'entrée du fluide allant de ladite extrémité proximale à ladite extrémité distale et à la récupération dudit fluide dans la région du corps adjacente à l'extrémité distale.

4. Cathéter de la revendication 1 ayant deux passages (40).

5. Cathéter de la revendication 1 ayant trois passages (50).

6. Cathéter de la revendication 1 ayant des moyens d'obturation de l'ouverture de l'utérus quand ce cathéter est en place dans ce dernier.

7. Cathéter de la revendication 6 dans lequel le moyen d'obturation de l'ouverture de l'utérus est un ballon annulaire gonflable (61).

8. Cathéter de la revendication 1 dans lequel une pluralité d'ouvertures distales (44) sont disposées selon une configuration en spirale le long de la partie extrême de ce cathéter.

9. Cathéter de la revendication 1 réalisé en matière transparente.

10. Cathéter de la revendication 1 réalisé en une matière qui est le polychlorure de vinyle, le polyéthlène ou le polytétrafluoroéthylène.

11. Cathéter de la revendication 1 dans lequel des repères sont placés à intervalles réguliers à l'extérieur de ce cathéter.

12. Cathéter de la revendication 1 ayant une flexibilité et une rigidité suffisantes pour lui permettre, quand il est tenu en laissant une longueur de trois centimètres comme partie extrême libre qui est pressée verticalement vers le bas contre une balance, de rester rectiligne sous une force de 0,42 N environ et de se courber en prenant un profil en C sous une force de 1,11 N à 2,22 N environ.

13. Cathéter de la revendication 1 ayant une flexibilité et une rigidité qui est mesurable par la tenue d'une tronçon de ce cathéter laissant trois centimètres comme partie extrême libre et qui après êtres tenu et en étant pressé vers le bas contre une balance avec approximativement un demi-centimètre orienté horizontalement sur la balance donne une lecture dans la gamme de environ 0,28 à 1,39 N quand ladite partie extrême libre dudit tronçon est courbée jusqu'à un profil incurvé avec une déviation de 45° approximativement.

14. Cathéter de la revendication 1 ayant une flexiblité et une rigidité telles qu'une longueur de trois centimètres de ce cathéter quand il est tenu en laissant trois centimètres comme partie extrême libre et pressé verticalement vers le bas contre une balance reste rectiligne jusqu'à une poussée vers le bas de 0,42 N environ et ce cathéter s'incurve ensuite sous une force additionnelle continue vers le bas.

15. Cathéter de la revendication 1 ayant une flexibilité et une rigidité telles qu'une longueur de trois centimètres de ce cathéter quand il est tenu en laissant trois centimètres comme partie extréme libre et qu'il est pressés verticalement vers le bas contre une balance cette partie extrême poussée contre la balance reste rectiligne et conserve son profil d'origine et ensuite s'incurve sous une posssée vers le bas allant de 1,11 à 2,22 N environ.

16. Cathéter de la revendication 1 ayant un diamètre extérieur compris entre 5 et 12 French environ.

17. Cathéter de la revendication 1 ayant une longueur comprise entre 150 et 200 mm environ.

18. Cathéter de la revendication 1 qui est un cathéter à triple passage (50) ayant un ballon annulaire (61) placé à un endroit éloigné de l'extrémité distale de ce cathéter, dans lequel lesdites ouvertures sont en avant dudit ballon, ce cathéter étant muni d'un moyen de remplissage du ballon permettant à ce dernier de se remplir et de se gonfler afin d'obturer le passage de liquide en provenance du tractus génital autrement que par une ouverture de l'extrémité distale du cathéter.

19. Cathéter de la revendication 1 qui est un cathéter à triple passage ayant un second passage de fluide et une seconde ouverture en communication avec ce second passage de fluide de sortie que ce cathéter parmet l'entrée de liquide dans l'utérus par l'un desdits passages de fluide et le sortie hors de l'utérus par l'autre desdits passages de fluide.

20. Cathéter de la revendication 1 ayant une variété d'ouvertures d'entrée et d'ouvertures de sortie pour permettre l'introduction de fluides et la récupération de fluides dans l'utérus.

FIG. I.

FIG. 2.

FIG. 3.

FIG. 4.

FIG. 5.

FIG. 6.

FIG. 7.

FIG. 8.

FIG. 9.

FIG. 10.

FIG. 11.

FIG. 12.

FIG. 13.